# EUROPÄISCHE PATENTSCHRIFT

(11) **EP 0 795 344 B1**
(45) Veröffentlichungstag und Bekanntmachung des Hinweises auf die Patenterteilung: **26.05.2004**
(21) Anmeldenummer: 97250075.5
(22) Anmeldetag: 14.03.1997
(51) Int. Cl.: A61N 1/372, A61N 1/05

(54) **Implantierbare Vorrichtung**
Implantable device
Dispositif implantable

(30) Priorität: 14.03.1996 DE 19611777
(43) Veröffentlichungstag der Anmeldung: 17.09.1997
(73) Patentinhaber: BIOTRONIK Mess- und Therapiegeräte GmbH & Co Ingenieurbüro Berlin, 12359 Berlin (DE)
(72) Erfinder: Schaldach, Max, Prof. Dr.-Ing., 91054 Erlangen (DE)
(74) Vertreter: Eisenführ, Speiser & Partner

(56) Entgegenhaltungen:
- EP-A- 0 596 625
- EP-A- 0 668 087
- DE-A- 2 453 840
- US-A- 4 258 724
- US-A- 4 444 207
- US-A- 5 086 787
- US-A- 5 385 579
- US-A- 5 447 533

## Beschreibung

Die Erfindung betrifft eine Vorrichtung der im Oberbegriff des Anspruchs 1 angegebenen Art.

Zur temporären Befestigung einer Stimulationsleitung am Herzmuskelgewebe wird die Stimulationselektrode entweder mit Hilfe einer mit der Vorhofwandung vernähten Halterung an der Außenwand einer Vorhofkammer des Herzens positioniert oder bei der Stimulation über eine der Herzkammern mit Hilfe eines mit seinem proximalen Ende an der Stimulationselektrode befestigten chirurgischen Fadens in das Gewebe der Herzkammer eingebracht.

Um zu verhindern, daß die temporär implantierte Stimulationselektrode bei normaler, durch die Herztätigkeit bedingte axiale Zugbelastung der Stimulationsleitung die ihr zugedachte Position verändert, ist ein Blockierungsmittel vorgesehen. Dieses besteht entweder aus der im wesentlichen in der Form an die Stimalationselektrode angepaßte Halterung, welche von außen an die relativ dünne Vorhofkammerwandung genäht ist oder das Blockierungsmittel wird durch einen besonders gestalteten Abschnitt im proximalen Bereich des chirurgischen Fadens gebildet, welcher den Reibwiderstand des chirurgischen Fadens gegenüber dem zu stimulierenden Gewebe an dieser Stelle erheblich erhöht.

Soll die temporär implantierte Stimulationselektrode nach einer bestimmten Zeit wieder von dem kontaktierten Gewebe getrennt werden, wird die Wirkung des jeweiligen Blockierungsmittels durch eine ruckartig erhöhte Zugbelastung der die Elektrode tragenden Stimulationsleitung aufgehoben. Die Leitung ist entgegengesetzt zur Einbringrichtung relativ leicht bewegbar und die Stimulationselektrode kann entfernt werden.

Die aus dem Stand der Technik bekannten Lösungen zur Fixierung der Position einer temporären Stimulationselektrode weisen jedoch den Nachteil auf, daß es beim Entfernen der Stimulationsleitung zu einer erheblichen Traumatisierung des kontaktierten Gewebes kommt, welche den Patienten körperlich stark belastet und den Heilungsprozeß in einem nicht vertretbaren Maß verzögert.

Ausgehend von den Mängeln des Standes der Technik liegt der Erfindung die Aufgabe zugrunde, eine Vorrichtung zur elektrischen Kontaktierung der eingangs genannten Gattung zu entwickeln, deren Ausgestaltung ein Entfernen einer temporär implantierten Stimulationselektrode in einem vorgebbaren Zeitraum nach der Implantation im wesentlichen ohne eine Traumatisierung des die Stimulationselektrode umgebenden Gewebes ermöglicht.

Diese Aufgabe wird mit den kennzeichnenden Merkmalen des Anspruchs 1 gelöst.

Die Erfindung schließt die Erkenntnis ein, daß die Traumatisierung des im Bereich der Stimulationselektrode befindlichen Gewebes im wesentlichen dadurch hervorgerufen wird, daß das Blockierungsmittel für die jeweilige Stimulationselektrode zur Erfüllung seiner Rückhaltefunktion derart ausgebildet ist, daß ein der Bewegung der Stimulationselektrode ein erhöhter mechanischer Widerstand entgegengesetzt wird. Dies ist entweder durch einen Formschluß zwischen Stimulationselektrode und einer mit dem zu stimulierenden Gewebe vernähten Halterung oder durch eine Erhöhung der Reibung bzw. des Widerstandes des chirurgischen Fadens bei seiner Bewegung gegenüber dem zu stimulierenden Gewebe verursacht.

Die eine ausreichende Fixierung der Stimulationselektrode sichernden Blockierungsmittel weisen funktionsbedingt eine relativ große Oberfläche auf, wodurch die Blockierungsmittel während des Implantationszeitraums eine mehr oder weniger feste Verbindung mit dem sie umgebenden Gewebe eingehen. Bei Entfernen der temporär implantierten Elektrode wird diese Verbindung gewaltsam aufgehoben und führt zu der unerwünschten Traumatisierung des Gewebes.

Gegenüber einem flexiblen Element weist ein starres Blokkierungsmittel einen besseren Rückhalteffekt auf, der durch die Resorption des Materials in der Körperflüssigkeit aber nach einiger Zeit aufgehoben wird, so daß die Rückhaltewirkung wieder völlig aufgehoben ist. Die sich damit einstellende kontrollierbare Differenz zwischen Haltewirkung und Freigabe weist damit ein Maximum auf.

Erfindungsgemäß bestehen die Blockierungsmittel aus einem zumindest teilweise in Körperflüssigkeit resorbierbaren Werkstoff, so daß eine temporär positionierte Stimulationselektrode nach einer bestimmten Zeitspanne bei erheblich reduzierter Traumatisierung des Gewebes aus dem Körper entfernt werden kann, da die eine ungewünschte Bewegung hemmende Wirkung der Blockiermittel durch Volumenverlust im wesentlichen aufgehoben.

Die Blockierungsmittel stellen ein starres Element dar und sind insbesondere nachträglich mit dem chirurgischen Faden oder mit der Stimulationselektrode verbindbar ausgebildet.

Besondere Vorteile sind bei der Wirkung der Blockierungsmittel erreichbar, wenn sie mittels zusätzlicher (insbeondere nicht resorbierbarer) elastischer Befestigungsmittel in Abhängigkeit von der Bewegungsrichtung der Stimulationselektrode ihre Gestalt dahingehend ändern, daß sie die Bewegung hemmen, wenn die Stimulationselektrode entgegen ihrer Einbringrichtung beim Positionieren an dem zu stimulierenden Gewebe bewegt werden soll.

Entsprechend einer bevorzugten Ausführungsform der Erfindung ist das Blockierungsmittel mit einer Halterung versehen, mit welcher die Stimulationselektrode von außen an dem zu stimulierenden Gewebe gesichert werden kann. Die Halterung ist im wesentlichen napfförmig ausgebildet und umgreift die Stimulationselektrode dadurch teilweise formschlüssig, wobei die nicht von der Halterung erfaßten Oberflächenabschnitte der Stimulationselektrode an das zu stimulierende Gewebe gepreßt werden. Zum Durchführen der Anschlußleitung der Stimulationselektrode und eines zum Positionieren der Elektrode verwendeten chirurgischen Fadens weist die Halterung im Randbereich in der Größe angepaßte, die Anschlußleitung und den Faden halbseitig umgreifende Ausnehmungen auf, so daß die Halterung mit ihrem Rand vollständig auf dem zu stimulierenden Gewebe aufliegt.

Besteht die Halterung oder bestehen zumindest die vorgenannten Randbereiche der napfförmigen Halterung aus einem resorbierbaren Werkstoff, so wird der für die Fixierung der Stimulationselektrode an einer bestimmten Gewebestelle erforderliche Formschluß nach einer bestimmten, vorgebbaren Zeitspanne aufgehoben. Dadurch kann die Stimulationselektrode in vorteilhafter Weise aus der Halterung herausgezogen werden, ohne daß eine stärkere Traumatisierung des Gewebes eintritt.

Die Halterung ist nach einer günstigen Weiterbildung der Erfindung mit verrundeten Kanten versehen. Dadurch wird einerseits das Einbringen der Stimulationselektrode in die an dem zu stimulierenden Gewebe durch Vernähen befestigte Halterung erleichtert und andererseits wird bei Entfernen der Stimulationselektrode die Möglichkeit einer Traumatisierung des Gewebes herabgesetzt, falls der biologische Abbau des resorbierbaren Werkstoffs noch nicht vollständig abgeschlossen ist.

Entsprechend einer anderen vorteilhaften Ausführungsform der Erfindung ist das aus einem resorbierbaren Kunststoff bestehenden Blockierungsmittel an dem proximalen Ende des zum Einbringen der Stimulationselektrode in das zu kontaktierende Gewebe benutzten chirurgischen Fadens vorgesehen.

Nach einer günstigen Weiterbildung der Erfindung ist das Blockierungsmittel als scheibenförmiges, aufsteckbares Element ausgebildet und weist einen sich im radial von außen nach innen erstreckten Schlitz auf. Um das Aufstecken des Blockierungsmittels auf den aus dem zu stimulierenden Gewebe herausragenden chirurgischen Faden zu erleichern, ist der Schlitz im peripheren Scheibenbereich konisch ausgebildet. Gleichzeitig sind die Schlitzkanten im übrigen Bereich, insbesondere im Bereich des Schlitzendes zueinander parallel geführt, um einen festen Sitz des Blockierungsmittels auf dem chirurgischen Faden zu sichern. Der Durchmesser des chirurgischen Fadens weist dazu einen größeren Wert auf als der Abstand der parallel zueinander geführten Schlitzkanten.

Das auf den chirurgischen Faden verschieblich ausgebildete Blockierungsmittel ersteckt sich erfindungsgemäß im wesentlichen quer zur Fadenrichtung. Das aufgesteckte Blokkierungsmittel kann bis an den Durchdringungspunkt des chirurgischen Fadens an der Gewebeoberfläche geschoben werden und liegt dort flächig auf dem Körpergewebe auf, so daß eine Veränderung der Position der Stimulationselektrode bei normaler Bewegung des stimulierten Gewebes wirksam unterbunden wird.

Entsprechend einer anderen Weiterbildung der Erfindung weist das resorbierbare Blockierungsmittel die Form einer auf den monofilen chirurgischen Faden aufschiebbaren Hülse auf, an welcher mindestens ein hakenförmiges Element vorgesehen ist. Das hakenförmige Element ist als eine sich im wesentlichen konisch verjüngende Lasche ausgebildet und überragt die Hülse in deren Längsrichtung. Die einzelnen Blockierungsmittel sind derart auf den chirurgischen Faden aufgeschoben, daß die Laschen der einzelnen Hülsen wechselseitig angeordnet sind. Die Laschen sind an der Hülse jeweils mit ihrem breiteren Ende befestigt und erstrecken sich dabei im wesentlichen parallel zur Fadenachse. Eine derartige Befestigung sichert in vorteilhafter Weise, daß sich die Laschen bei einer Fadenbewegung in Einbringungsrichtung des Fadens beim Positionieren der Stimulationselektrode in das zu kontaktierende Gewebe an den Faden anschmiegen und dabei nur einen geringen Reibungswiderstand bewirken. Wird der chirurgische Faden nach Positionieren der Stimulationselektrode zugentspannt, so geht er aufgrund seiner Materialeigenschaften aus der vollkommen gestreckten in eine leicht gewellte Form über. Dabei erfolgt ein Abspreizen der einzelnen Laschen relativ zur Fadenachse. Bei einer Zugwirkung auf das Anschlußkabel einer derart temporär implantierten Stimulationselektrode entgegengesetzt zur Einbringrichtung übernehmen die einzelnen Laschen die Funktion eines Widerhakens und wirken einer Bewegung aufgrund der Zugkraft blockierend entgegen.

Die bewegungshemmende Wirkung der Blockiermittel tritt entsprechend einer vorteilhaften Weiterbildung besonders schnell ein, wenn die Laschen an die Hülse in einer elastischen Lagerung im Bezug auf die Achse des chirurgischen Fadens nach außen schwenkbar angelenkt sind.

Nach einem vorgebbaren, durch den eingesetzten Werkstoff bestimmten Zeitraum sind die resorbierbaren Blockierungsmittel soweit abgebaut, daß sie ihre Funktion als Widerhaken nicht mehr aufrechterhalten können. Die temporär implantierte Stimulationselektrode kann dann ohne Traumatisierung des Gewebes entgegengesetzt zu ihrer Einbringrichtung aus dem Gewebe gezogen werden.

Das Fixieren des hülsenförmigen Blockierungsmittels aus resorbierbarem Material beim Aufschieben auf das proximale Ende des monofilen chirurgischen Fadens erfolgt durch Haftreibung, welche mittels maßlicher Anpassung des Faden-und des Hülsendurchmessers in der erforderlichen Größe erzeugt wird.

Bei Verlängerung der Hülse sind an der äußeren Hülsenwand mehrere laschenförmige, sich konisch verjüngende Elemente derart anordenbar, daß sie sich bei Einbringen der Stimulationselektrode in das zu kontaktierende Gewebe an die Hülsenwandung anschmiegen und eine Bewegung der Stimulationselektrode in entgegengesetzter Richtung als Widerhaken blokkieren. Die laschenförmigen Elemente sind in günstiger Weise wechselständig oder paarweise gegenständig an der Wandung des hülsenförmigen Blockierungsmittels angeordnet. Die Hakenwirkung wird noch verstärkt, wenn die paarweise gegenständig befestigten Laschen in jeder Befestigungsebene gegeneinander um 90° paarweise versetzt werden. Nach Ablauf der zur Resorption erforder-lichen Zeitspanne kann die Elektrode ohne Traumatisierung des Gewebes entfernt werden.

Nach einer zusätzlichen Weiterbildung der Erfindung ist dem chirurgischen Faden ein stabförmiges Blockierungsmittel zugeordnet, welches durch Umwickeln mit dem Faden verbunden ist. In dem stabförmigen Blockierungsmittel ist eine Querbohrung vorgesehen, durch welche der chirurgische Faden vor dem Umwickeln des Blockierungsmittels geführt wird. Das Umwickeln erfolgt derart, daß sich die Achse des stabförmigen Blockierungsmittels im wesentlichen senkrecht zur Zugrichtung beim Entfernen der Stimulationselektrode erstreckt. Nach vollständigem Resorbieren des stabförmigen Blockierungsmittels ist das distale Ende des chirurgischen Fadens freigegeben und die temporär implantierte Stimulationselektrode kann ohne Traumatisierung des kontaktierten Gewebes entfernt werden.

Entsprechend einer weiteren Ausführungsform der Erfindung ist ein vollständig aus resorbierbaren Werkstoff bestehender chirurgischer Faden vorgesehen, der in seinem proximalen Bereich als Blockierungsmittel einen wendelförmigen Abschnitt aufweist. Diese Wendel wird beim Einbringen der Stimulationselektrode durch die auf den chirurgischen Faden wirkende Zugkraft gestreckt. Sie nimmt ihre ursprüngliche Form im wesentlichen wieder an, wenn die Stimulationselektrode positioniert ist und fixiert somit die Elektrode an der vorgesehenen Position. Die Stimulationselektrode läßt sich ohne Traumatisierung des Gewebes entfernen, wenn das Blockierungsmittel bzw. der gesamte chirurgische Faden resorbiert worden ist.

Gute Ergebnisse bei der Lagesicherung einer temporär positionierten Stimulationselektrode sind mit Blockierungsmitteln aus einem resorbierbaren osteosynthetischen Werkstoff, beispielsweise aus Polyglycol-Säure, erzielbar.

Entsprechend einer zusätzlichen Ausführungsform der Erfindung besteht der chirurgische Faden aus einem zugfesten, resorbierbaren Werkstoff. Das Blockierungsmittel wird durch Formgebung des Fadens erzeugt. Eine aus-reichende Bewegungshemmung ist durch einen wendelförmig ausgebildeten Fadenabschnitt als Blockierungsmittel gegeben. Das Blockierungsmittel ist in vorteilhafter Weise am proximalen Ende des chirurgischen Fadens, d.h. in der Nähe der Verbindungsstelle des Fadens mit der Stimulationselektrode, vorgesehen. Aufgrund der Eigenelastizität des Fadenmaterials wird das Blockierungsmittel beim Einbringen der Stimulationselektrode überdehnt und setzt der Bewegung einen geringen Widerstand entgegen. Bei Zugentlastung auf der distalen Elektrodenseite nimmt das Blockierungmittel seine ursprüngliche Wendelform an und hemmt eine Bewegung entgegengesetzt zur Einbringrichtung der Stimulationselektrode.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine Draufsicht auf einen zu kontaktierenden Gewebeabschnitt mit einer bevorzugten Ausführungsform der Erfindung,
Figur 2 die Ansicht eines Schnittes längs der Linie A...A gemäß Figur 1,
Figur 3 eine andere vorteilhafte Ausführungsform der Erfindung,
Figur 4 die Schnittdarstellung eines kontaktierten Gewebebereichs mit einer erfindungsgemäßen Vorrichtung nach Figur 3,
Figur 5 die Schnittdarstellung eines kontaktierten Gewebes mit einer günstigen Weiterbildung der in den Figuren 3 und 4 gezeigten Erfindung,
Figur 6 die Schnittdarstellung eines kontaktierten Gewebebereichs mit einer weiterer Ausführungsform der Erfindung während der Einbringphase der Stimulationselektrode,
Figur 6a die Schnittdarstellung des kontaktierten Gewebereiches gemäß Figur 5 nach Implantation der Elektrode,
Figur 6b die Darstellung der Einzelheit E gemäß Figur 6a,
Figur 7 die Darstellung einer günstigen Weiterbildung der in Figur 6 gezeigten Erfindung sowie
Figur 8 die Darstellung eines Schnittes längs der Linie B...B gemäß Figur 7.

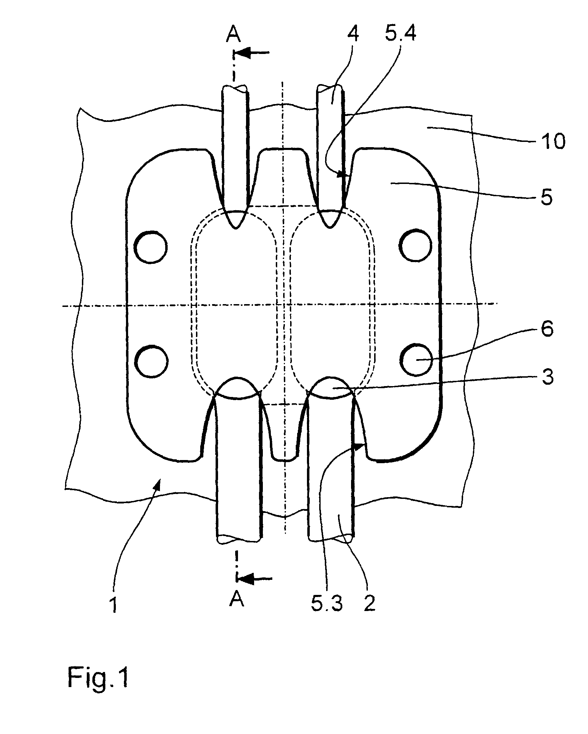

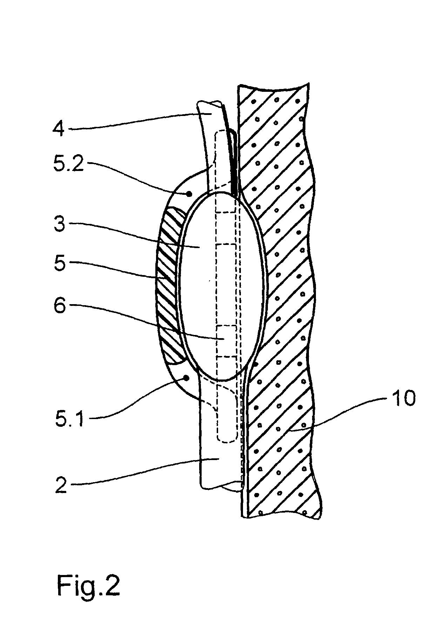

Figur 1 zeigt in Draufsicht auf eine Vorrichtung 1 mit einer Halterung 5, durch welche zwei temporär implantierte Stimulationselektroden 3 an dem Gewebe eines Herzmuskels 10 zwecks Kontaktierung eines (nicht dargestellten) medizinischen Geräts fixiert werden. In Figur 2 ist die Ansicht des Schnittes längs der Linie A...A entsprechend Figur 1 dargestellt.

Die aus einem resorbierbaren Material bestehende Halterung 5 ist napfförmig ausgebildet und mit dem Gewebe 10 unter Nutzung der Durchbrüche 6 durch Vernähen befestigt.

Die Stimulationsleitungen weisen jeweils eine kuglig ausgebildete Elektrode 3 auf, welche proximal mit dem isolierten Leiter 2 und distal mit einem chirurgischen Faden 4 verbunden ist. Unter Nutzung des Fadens 4 werden die Stimulationselektroden 3 in den Freiraum zwischen dem Herzmuskelgewebe 10 und der Halterung 5 gezogen, wobei die Halterung 5 die Elektroden halbseitig formschlüssig umgreift und zwecks Kontaktierung gegen das nachgiebige Herzmuskelgewebe 10 preßt. Die Halterung 5 weist an ihrem Rand jeweils zwei gleichartige, bogenförmig begrenzte und einander gegenüberliegende Ausnehmungen 5.3 und 5.4 auf, welche das Einbringen der Stimulationselektroden 3 in den Freiraum zwischen Gewebe 10 und Halterung 5 wesentlich erleichtern. Es ist gleichermaßen für ein bequemes Einbringen von Vorteil, wenn alle Kanten der Halterung 5 verrundet ausgebildet sind.

Die derart positionierten Stimulationselektroden 3 werden durch die Randabschnitte 5.1 und 5.2 der Halterung 5 - insbesondere im Bereich der Ausnehmungen 5.3 und 5.4 -gegen Herausgleiten aus der Halterung 5 fixiert, wenn auf die Elektrode 3 bzw. auf die Stimulationsleitung 2 in axialer Richtung eine Zugkraft wirkt, welche beispiels-weise durch die funktionsgemäße Bewegung des Herzmuskels 10 erzeugt wird.

Da die Halterung 5 oder zumindest der Halterungsrand aus einem resorbierbaren Werkstoff besteht, wird durch den zeitlich fortschreitenden Materialabbau insbesondere im Bereich der Ausnehmungen 5.3 am Rand der Halterung 5 - die axiale Rückhaltwirkung des Randes gegenüber den Stimulationselektroden 3 stetig verringert, so daß die Stimulationselektroden nach Ablauf einer vorgebbaren Zeitspanne (Auswahl eines Werkstoffs mit entsprechender Abbauzeit) aus der Halterung 5 entfernt werden kann, ohne daß es zu einer Traumatisierung des Gewebes 10 kommt.

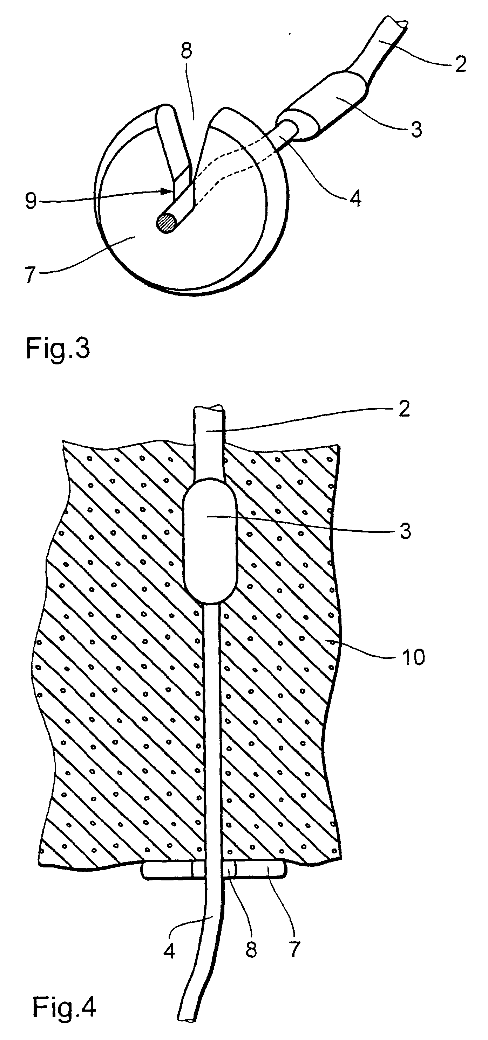

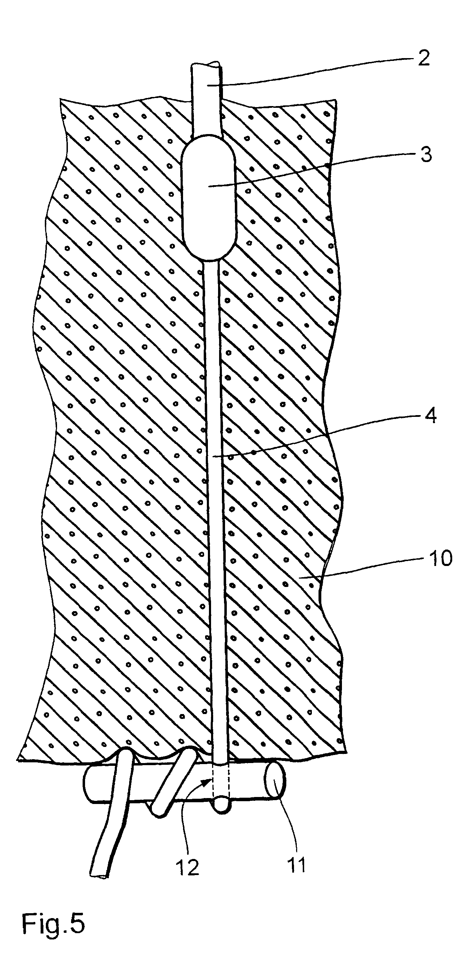

In den Figuren 3 und 4 ist eine Ausführungsform der Erfindung in perspektivischer Darstellung bzw. in Darstellung eines Schnittes durch ein mittels einer temporären Stimulationselektrode 3 kontaktiertes Herzmuskelgewebe 10 gezeigt.

Als Blockierungsmittel ist eine kreisförmige Scheibe 7 vorgesehen. Die Scheibe 7 ist mit einem sich radial erstrekkenden, peripher keilförmig geöffneten Schlitz 8 versehen. Dadurch kann das Blockierungsmittel bequem auf das distale, aus dem zu kontaktierenden Herzmuskelgewebe 10 herausgeführte Ende des chirurgischen Fadens 4 aufgesteckt werden, wobei sich das Blockierungsmittel 7 nach Verschieben auf dem Faden 4 auf der Außenseite des Gewebes 10 abstützt. Um einen festen Sitz des scheibenförmigen Blockierungsmittels 7 auf dem chirurgischen Faden 4 zu sichern, weist der dem Scheibenmittelpunkt zugewandte Abschnitt 9 des Schlitzes 8 parallel geführte Kanten auf. Der daraus resultierende Formschluß zwischen Faden 4 und Blockierungsmittel 7 wird erst aufgehoben, wenn der resorbierbare Werkstoff weitgehend genug abgebaut worden ist. Danach ist die temporär implantierte Stimulationselektrode 3 durch Zugbelastung der Anschlußleitung 2 ohne merkliche Traumatisierung des Herzmuskelgewebes 10 entfernbar.

Der feste Sitz des Blockierungsmittels 7 wird durch einen Schlitz 8 erreicht, dessen Kantenabstand im Bereich 9 geringer ist als der Durchmesser des chirurgischen Fadens 4.

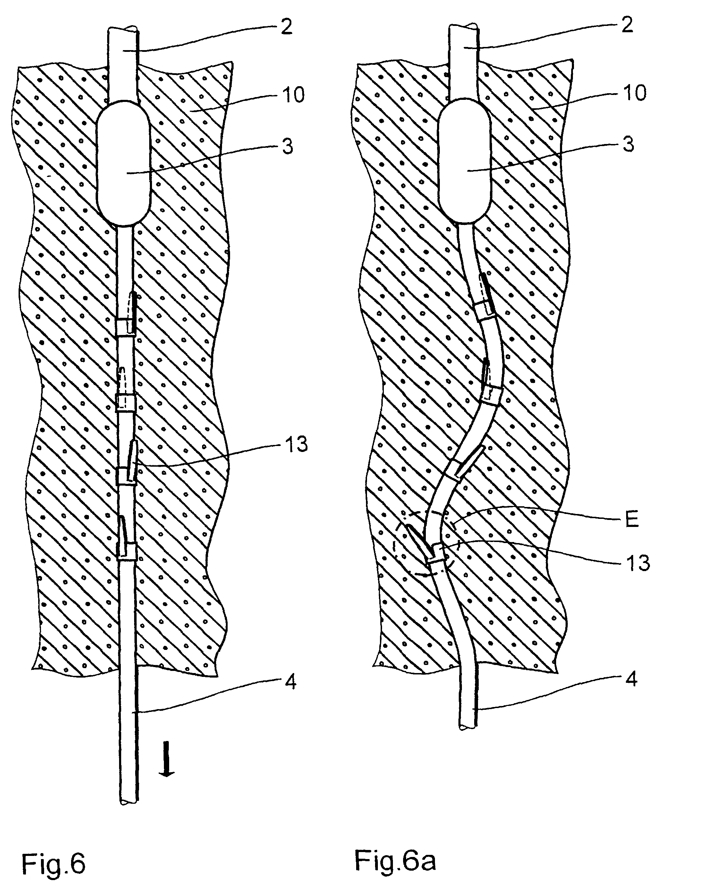

Die Schnittdarstellung gemäß Figur 5 zeigt eine in einem Herzmuskelgewebe 10 temporär implantierte Stimulationselektrode 3 mit einer Anschlußleitung 2, welche unter Verwendung eines chirurgischen Nähfadens 4 in dem zu stimulierenden Gewebe 10 plaziert wird. Zum Fixieren der vorgesehenen Position der Stimulationselektrode 3 ist ein stabförmiges aus einem resorbierbaren Werkstoff bestehendes Blockierungsmittel 11 vorgesehen. Das Blockierungsmittel 11 weist eine Querbohrung 12 auf, durch welche das distale, aus dem Gewebe 10 herausragende Ende des chirurgischen Fadens 4 geführt ist. Nach Umwickeln mit dem Fadenende stützt sich das stabförmige Blockierungsmittel 11 an der Gewebeaußenseite ab, wobei sich die Längsachse des Blockierungsmittels 11 im wesentlichen senkrecht zur Achse des chirurgischen Fadens 4 erstreckt. Bedingt durch Haftreibung und Formschluß ergibt sich bereits bei zwei bis drei Fadenwindungen ein ausreichend fester Sitz des Blockierungsmittels 11. Nach Abbau des resorbierbaren Blockierungsmittels 11 wird der chirurgische Faden 4 freigegeben und die Stimulationelektrode 3 ist durch Zug an der Anschlußleitung 2 problemlos entfernbar.

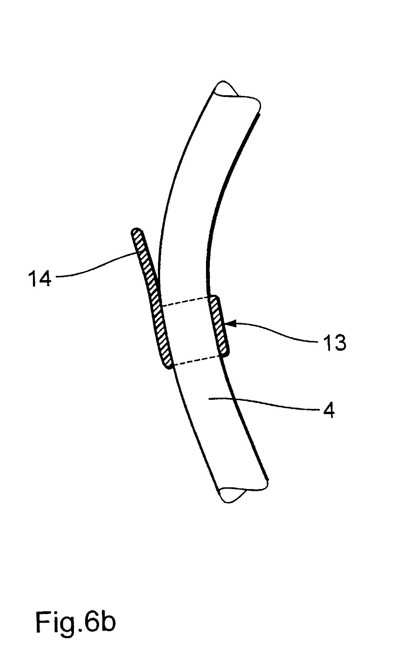

In den Figuren 6, 6a und 6b ist eine Vorrichtung mit auf den chirurgischen Faden 4 aufschiebbaren Blockierungsmitteln 15 dargestellt. Die hülsenförmig ausgebildeten Blokkie-rungsmittel 15 sind am proximalen Ende des chirurgischen Fadens 4 angeordnet und weisen jeweils ein Halteelement 14 auf, welches sich beim Einbringen des chirurgischen Fadens 4 in das Gewebe 10 in Richtung der Hülsenachse anschmiegt. Das Halteelement 14 weist die Form einer sich konisch verjüngenden Lasche auf, welche mit dem breiteren Ende an der Hülse 13 befestigt ist und diese in der Länge überragt (vergleiche Figur 6b).

Die Blockierungsmittel 15 sind derart in Reihe auf dem Faden angeordnet, daß ihre laschenartigen Halteelemente 14 im wesentlichen wechselständig angeordnet sind, wobei sie sich im wesentlichen parallel zur Achse des chirurgischen Fadens 4 erstrecken. Beim Einbringen der Stimulationselektrode 3 in das Herzmuskelgewebe 10 wird der chirurgische Faden in Pfeilrichtung gezogen (Figur 6) und die Halteelemente 14 der Blockierungsmittel 15 schmiegen sich an die Hülsen- bzw. Fadenachse an. Dadurch entsteht beim Durchdringen des Herzmuskelgewebes nur ein geringer zusätzlicher Reibungswiderstand durch die Blockierungsmittel 15. Ist die Stimulationselektrode 3 plaziert, so wirkt auf den chirurgischen Faden 4 keine Zugspannung (Figur 6a) und der Faden 4 orientiert sich aufgrund seiner Eigenspannung bogenförmig innerhalb des Gewebes 10. Dadurch werden die Halteelemente 14 um ein bestimmtes Maß abgespreizt (Figur 6b). Die Halteelemente 14 erstrecken sich jetzt im wesentlichen tangential zur Achse des chirurgischen Fadens und wirken somit entgegengesetzt der Einbringrichtung des Fadens 4 als Widerhaken. Die gleiche Effekt wird durch eine entgegengesetzt zur Einbringrichtung des Fadens wirkende Zugkraft auf die Elektrodenleitung erreicht.

Die blockierende Wirkung der Halteelemente 14 wird mit zunehmender Zeit durch den Materialabbau des resorbierbaren Werkstoffs immer mehr reduziert, so daß die Stimulationselektrode 3 nach einer - durch Werkstoffauswahl für das Blockierungsmittel festgelegt - vorgebbaren Zeitspanne ohne Widerhalt aus dem Herzmuskelgewebe 10 entfernt werden kann.

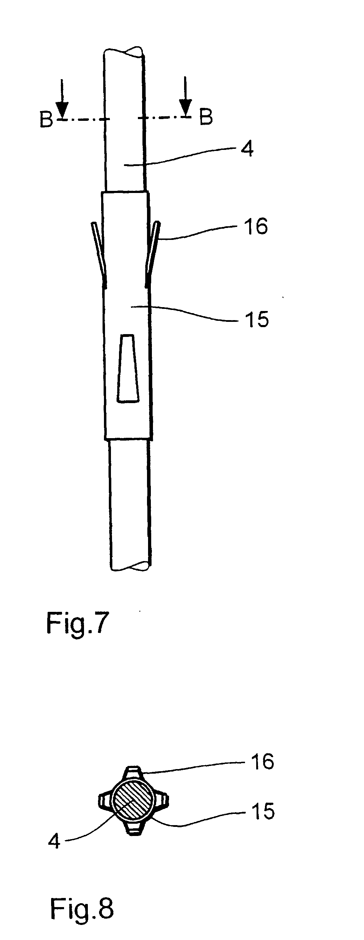

Das in der Figur 7 in Seitenansicht dargestellte resorbierbare Blockierungsmittel 15 weist die Form einer länglichen Hülse auf und ist auf den proximalen Bereich des chirurgischen Fadens 4 aufgeschoben. Die Halteelemente 16 sind paarweise in zwei übereinanderliegenden Ebenen an der Außenwandung der Hülse angeformt. Die Halteelemente 16 sind als schmale, sich konisch verjüngende Lasche ausgebildet und mit ihrem breiten Ende an der Hülse befestigt. Die Halteelemente 16 sind in jeder Ebene gegen-ständig angeordnet, wobei sich die Halteelemente der einen Ebene in einer um 90° versetzten Position gegenüber den Halteelementen der anderen Ebene befinden. Figur 8 verdeutlicht durch Darstellung der Ansicht eines Schnittes längs der Linie B...B gemäß Figur 7 die Anordnung der einzelnen Halteelementpaare, deren Achse sich unter einem spitzen Winkel geringer Größe zur Achse des chirurgischen Fadens 4 erstreckt. Wird der chirurgische Faden 4 zwecks Positionierung einer (nicht dargestellten) temporären Stimulationselektrode durch das zu stimulierende Herzmuskelgewebe gezogen, schmiegen sich die Halteelemente 16 unter Verringerung des Reibungswiderstandes des Fadens an die Hülsenwandung an. Bei einer Bewegung des Fadens 4 in entgegengesetzter Richtung werden die Halteelemente 16 abgespreizt und wirken als Widerhaken dieser Bewegung entgegen.

Diese Eigenschaft wird zur Fixierung einer temporär in ein Herzmuskelgewebe implantierten Stimulationselektrode genutzt, welche nach Ablauf einer festgelegten Zeitspanne, nach der die Rückhaltewirkung der Widerhaken durch Materialabbau aufgehoben ist, ohne Traumatisierung des Gewebes entfernbar ist.

Die Abspreizneigung der in den Figuren 6, 6a, 6b, 7 und 8 gezeigten Halteelemente 14, 18 ist auf einfache Weise vergrößerbar, indem die Haltemittel in einer elastischen Lagerung an die jeweilige Hülse 13, 16 des Blockiermittels 15, 17 schwenkbar angelenkt sind.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

Es ist ersichtlich, daß das starre resorbierbare Blockierungsmittel in vielerlei Varianten sowohl einer implantierbaren Elektrode als auch an deren Zuleitung sowie ein einem mit der Elektrode verbundenen Befestigungsmittel vorgesehen sein kann.

## Patentansprüche

1. Implantierbare Vorrichtung zur mindestens temporären elektrischen Kontaktierung von Körpergewebe (10), vorzugsweise des menschlichen Herzmuskels, mittels einer Stimulationselektrode, die entweder mit Hilfe einer mit der Vorhofwandung vernähten Halterung an der Außenwand einer Vorhofkammer des Herzens zu positionieren ist oder bei der Stimulation über eine der Herzkammern mit Hilfe eines mit seinem proximalen Ende an der Stimulationselektrode befestigten chirurgischen Fadens in das Gewebe der Herzkammer einzubringen ist,
- mit einer Leitung (2), deren proximales Ende mit einem medizinischen Gerät verbindbar ist und an deren distalem Ende die Stimulationselektrode (3) fest angebracht ist,
- mit einem Blockierungsmittel zum Festhalten der Stimulationselektrode (3) in ihrer das Körpergewebe kontaktierenden Position gegen eine über die Leitung aufgebrachte Zugkraft,
**dadurch gekennzeichnet,**
**dass** das im wesentlichen starre Blockierungsmittel (5, 7, 11, 15, 17) mindestens teilweise aus einem in Körperflüssigkeit resorbierbaren Werkstoff besteht.

2. Vorrichtung nach Anspruch 1, **dadurch gekennzeichnet, daß** das Blockierungsmittel (15, 17) gegen die Richtung der Zugkraft geneigt ausgestaltet ist.

3. Vorrichtung nach Anspruch 1, **dadurch gekennzeichnet, daß** das Blockierungsmittel (5, 7, 11) mit der Stimulationselektrode oder dem chirurgischen Faden (4) mittels einer auf diese insbesondere aufsteck- oder aufschiebbaren Halterung verbindbar ausgebildet ist.

4. Vorrichtung nach Anspruch 3, **dadurch gekennzeichnet, daß** die Halterung (5) die Stimulationselektrode (3) oder den Faden in Form einer Hülse im wesentlichen formschlüssig umgreift.

5. Vorrichtung nach Anspruch 3, **dadurch gekennzeichnet, daß** die Halterung (5) im wesentlichen napfförmig ausgebildet ist, wobei mindestens Teile (5.1, 5.2) von dessen Rand aus einem resorbierbaren Werkstoff bestehen.

6. Vorrichtung nach Anspruch 5, **dadurch gekennzeichnet, daß** in dem Rand des Napfes Ausnehmungen (5.3, 5.4) vorgesehen sind, die einen Durchlaß bilden und die Stimulationselektrode (2) bzw. den chirurgischen Faden (4) mindestens teilweise umgreifen.

7. Vorrichtung nach Anspruch 1, **dadurch gekennzeichnet, daß** das Blockierungsmittel (7) scheibenförmig ausgebildet und insbesondere einen sich radial erstreckenden, nach außen geöffneten Schlitz (8) oder eine Lochung zur Durchführung eines Fadens aufweist.

8. Vorrichtung nach Anspruch 1, **dadurch gekennzeichnet, daß** das Halteelement (14) die Form einer sich konisch verjüngenden Lasche aufweist, welche mit dem breiteren Ende an einer Hülse (13) befestigt ist und diese in der Länge überragt.

9. Vorrichtung nach Anspruch 4, **dadurch gekennzeichnet, daß** das Halteelement (14, 18) derart elastisch mit einer Hülse (13, 16) verbunden ist, daß sich das Blockierungsmittel (14, 18) einerseits beim Einführen der Elektrode (4) in das Gewebe (10) an die Fadenoberfläche anschmiegt und andererseits in Form eines Widerhakens abgespreizt wird, wenn auf den Faden (4) oder eine Zugkraft in Auszugsrichtung wirkt.

10. Vorrichtung nach Anspruch 1, **dadurch gekennzeichnet, daß** mehrere Blockierungsmittel (15) in einer Reihe, insbesondere paarweise gegenständig an einer Hülse (16) oder um im wesentlichen 90° gegeneinander versetzt angeordnet sind.

11. Vorrichtung nach Anspruch 1, **dadurch gekennzeichnet, daß** als resorbierbarer Werkstoff eine Polyglycol-Säure vorgesehen ist.

## Claims

1. Implantable device for at least temporary electrical contact with body tissue (10), preferably with the human heart muscle, by means of a stimulation electrode, which is either to be positioned on the outer wall of a vestibule of the heart with the aid of a holder sewn to the vestibule wall, or, on stimulation via one of the heart's chambers, is to be introduced into the tissue of the heart chamber with the aid of surgical thread fastened by its proximal end to the stimulation electrode, comprising a lead (2), of which the proximal end can be connected to a medical instrument and to the distal end of which the stimulation electrode (3) is securely attached, comprising blocking means for holding the stimulation electrode (3) in its position of contact with the body tissue, counter to a pulling force exerted via the lead, **characterised in that** the substantially rigid blocking means (5, 7, 11, 15, 17) consists at least partially of a material that can be resorbed into body fluid.

2. Device according to claim 1, **characterised in that** the blocking means (15, 17) is designed such that it is inclined counter to the direction of the pulling force.

3. Device according to claim 1, **characterised in that** the blocking means (5, 7, 11) can be connected to the stimulation electrode or the surgical thread (4) by means of a holder which, in particular, can be fitted or pushed onto the electrode.

4. Device according to claim 3, **characterised in that** the holder (5) encircles the stimulation electrode (3) or the thread in the form of a sleeve with substantially interlocking fit.

5. Device according to claim 3, **characterised in that** the holder (5) has a substantially cup-shaped design, at least portions (5.1, 5.2) of the edge thereof consisting of a material that can be reabsorbed.

6. Device according to claim 5, **characterised in that** recesses (5.3, 5.4) are provided in the cup edge, which form a passage and at least partially encircle the stimulation electrode (2) or the surgical thread (4).

7. Device according to claim 1, **characterised in that** the blocking means (7) has a disc-shaped design and, in particular, has a radially extending slot (8) that opens outward, or a hole, for guiding through a thread.

8. Device according to claim 1, **characterised in that** the holding element (14) has the shape of a conically tapered strap, which is fastened with the wider end to a sleeve (13) and projects over the sleeve lengthwise.

9. Device according to claim 4, **characterised in that** the holding element (14, 18) is connected elastically to a sleeve (13, 16) such that, on the one hand, the blocking means (14, 18) will conform to the surface of the thread when the electrode (4) is inserted into the tissue (10), and, on the other hand, is spread in the form of a barb if a counter pulling force is exerted on the thread (4).

10. Device according to claim 1, **characterised in that** a plurality of blocking means (15) are arranged in a row, in particular in opposing pairs on a sleeve (16) or are arranged mutually offset by substantially 90°.

11. Device according to claim 1, **characterised in that** a polyglycol acid is provided as the material that can be resorbed.

## Revendications

1. Dispositif implantable pour la mise en contact électrique au moins temporaire de tissu corporel (10), de préférence du muscle cardiaque humain, au moyen d'une électrode de stimulation, qui doit être positionné à l'aide d'une fixation cousue avec la paroi de l'oreillette sur la paroi externe d'une cavité auriculaire du coeur ou qui doit être introduit dans le tissu de la cavité cardiaque lors de la stimulation par le biais de l'une des cavités cardiaques au moyen d'un fil chirurgical fixé par son extrémité proximale à l'électrode de stimulation,
- avec un conducteur (2) dont l'extrémité proximale peut être reliée à un appareil médical et à l'extrémité distale duquel est disposée de manière fixe l'électrode de stimulation (3),
- avec un moyen de blocage pour fixer l'électrode de stimulation (3) dans sa position en contact avec le tissu corporel à l'encontre d'une force de traction appliquée par le biais du conducteur,
**caractérisé en ce que** le moyen de blocage (5, 7, 11, 15, 17) sensiblement rigide consiste au moins en partie en un matériau résorbable dans les liquides biologiques.

2. Dispositif selon la revendication 1 **caractérisé en ce que** le moyen de blocage (15, 17) est agencé de manière inclinée par rapport à la direction de la force de traction.

3. Dispositif selon la revendication 1 **caractérisé en ce que** le moyen de blocage (5, 7, 11) est agencé de manière à pouvoir être relié à l'électrode de stimulation ou au fil chirurgical (4) au moyen d'une fixation qui peut en particulier être appliquée ou enfilée dessus.

4. Dispositif selon la revendication 3 **caractérisé en ce que** la fixation (5) entoure l'électrode de stimulation (3) ou le fil de manière sensiblement à assemblage de forme sous forme d'une gaine.

5. Dispositif selon la revendication 3 **caractérisé en ce que** la fixation (5) est agencée sensiblement en forme de cuvette où au moins des parties (5.1, 5.2) de son bord consistent en un matériau résorbable.

6. Dispositif selon la revendication 5 **caractérisé en ce qu'**il est prévu dans le bord de la cuvette des évidements (5.3, 5.4) qui forment un passage et qui entourent au moins partiellement l'électrode de stimulation (2) ou le fil chirurgical (4).

7. Dispositif selon la revendication 1 **caractérisé en ce que** le moyen de blocage (7) est agencé en forme de disque et comporte en particulier une fente (8) ouverte vers l'extérieur, qui s'étend radialement, ou un perçage pour le passage d'un fil.

8. Dispositif selon la revendication 1 **caractérisé en ce que** l'élément de fixation (14) présente la forme d'un collier qui se rétrécit coniquement, qui est fixé par l'extrémité plus large à une gaine (13) qu'il dépasse en longueur.

9. Dispositif selon la revendication 4 **caractérisé en ce que** l'élément de fixation (14, 18) est relié élastiquement avec une gaine (13, 16) de telle sorte que le moyen de blocage (14, 18) d'une part s'adapte à la surface du fil lors de l'insertion de l'électrode (4) dans le tissu (10) et d'autre part s'épanouit sous forme d'une barbe quand une force de traction agit dans la direction de retrait sur le fil (4).

10. Dispositif selon la revendication 1 **caractérisé en ce que** plusieurs moyens de blocage (15) sont disposés en une série, en particulier de manière opposée par paires dans une gaine (16) ou de manière mutuellement décalée de sensiblement 90°.

11. Dispositif selon la revendication 1 **caractérisé en ce qu'**un poly(acide glycolique) est prévu comme matériau résorbable.
